# FASCICULE DE BREVET EUROPEEN

(11) **EP 3 060 465 B1**
(45) Date de publication et mention de la délivrance du brevet: **11.04.2018**
(21) Numéro de dépôt: 14786894.7
(22) Date de dépôt: 20.10.2014
(51) Int. Cl.: B63B 22/04, B63B 22/18, G01N 33/18

(54) **STATION OFFSHORE DE MESURE ET DE COLLECTE DE DONNEES EN MILIEU SOUS-MARIN**
OFFSHORE-STATION ZUR MESSUNG UND ERHEBUNG VON DATEN IN EINER UNTERWASSERUMGEBUNG
OFFSHORE STATION FOR MEASURING AND COLLECTING DATA IN AN UNDERWATER ENVIRONMENT

(30) Priorité: 21.10.2013 FR 1360256
(43) Date de publication de la demande: 31.08.2016
(73) Titulaire: Universite de Nantes, 44000 Nantes (FR); Centre National de la Recherche Scientifique (C.N.R.S.), 75794 Paris Cedex 16 (FR)
(72) Inventeur: SCHOEFS, Franck, F-44240 Sucé sur Erdre (FR); AMERYOUN, Hamed, F-44000 Nantes (FR)
(74) Mandataire: Novagraaf Technologies
(86) Numéro de dépôt international: PCT/EP2014/072467
(87) Numéro de publication internationale: WO 2015/059107

(56) Documents cités:
- KR-A- 20120 073 100
- US-A1- 2012 090 385
- Adrias Q Araceli ET AL: "Subsea coatings research and the challenges of the deep sea", ASIA PACIFIC COATINGS JOURNAL, 30 mai 2010 (2010-05-30), XP055127163, Extrait de l'Internet: URL:http://www.poseidonsciences.com/APCJ_s ubsea_immersion_biofouling_barnacles_Adria s_Matias.pdf [extrait le 2014-07-07]

## Description

### Domaine technique

La présente invention se rapporte au domaine des observatoires sous-marins. L'invention concerne plus particulièrement une station offshore de mesure de données et de collecte de bio-salissures marines dans un milieu sous-marin.

### Etat de la technique

Lorsqu'une structure offshore est immergée dans l'eau de mer, elle est rapidement soumise à des mécanismes de dégradation comme la corrosion et la bio-colonisation. Cette dernière représente, dans le cadre des Energies Marines Renouvelables (EMR), un enjeu important. Elle correspond à une couverture de la structure par des organismes marins animaux (moules, balanes, ..) et végétaux (algues, ...). Ces organismes et ces végétaux peuvent dégrader le matériau et sont la cause d'une surcharge en masse et hydrodynamique sur la structure.

Leur croissance est un phénomène complexe et il reste encore largement incompris. La localisation géographique, la distance de la côte, les vagues, les courants d'eau, la disponibilité des nutriments, les paramètres physicochimiques de l'eau de mer, la présence des systèmes de protection, l'activité humaine, et la date d'installation des plateformes en relation avec la date de libération des spores et les larves ont une incidence sur la nature et le taux de croissance des bio-salissures.
Des mesures récentes montrent que l'épaisseur de la croissance marine peut être considérable (supérieure à 150 mm) selon le lieu géographique du site d'implantation. Elles peuvent avoir un impact significatif sur la logistique et le coût des inspections structurelles et des opérations de maintenance et nettoyage.
D'un point de vue global, il faut savoir qu'en général, l'entretien d'une structure offshore est plus coûteux que sa fabrication. Dans le cas des EMR où le coût du kWh doit être très compétitif et le mieux maîtrisé possible, ce coût est très impactant, ce qui différencie notablement ce secteur du secteur pétrolier dit 'oil and gas'.
Il existe donc un besoin de recueillir des données sur la colonisation des salissures marines dans les champs offshores. Il s'agit alors d'instrumenter un site sous marin afin de recueillir des informations sur la dégradation et/ou la bio-colonisation d'une structure offshore au cours du temps, de préférence plusieurs années avant l'implantation de structures de production d'électricité ou de stockage d'énergie. Un exemple d'une structure sous marine de collecte de bio-salissure est divulgué par l'article "Subsea coatings research and the challenges of the deep sea" dans le "Asia Pacific Coatings Journal" d'Avril Mai 2010. (www.poseidonsciences.com/ APCJ_subsea_immersion_biofouling_barnacles_Adrias_Matias.pdf)

### Résumé de l'invention

Un objet de l'invention est de proposer une station de mesure permettant de recueillir des données pour une meilleure prévision des mécanismes de dégradations sur un site, notamment par des bio-salissures, afin de programmer l'entretien nécessaire d'une structure offshore qui serait implantée sur ce site.

Un autre objet de l'invention est de proposer une station de mesure qui soit peu coûteuse à fabriquer, facile à transporter, à mettre en place sur un site particulier et à démanteler par des moyens conventionnels disponibles dans les ports de pêche et qui puisse être facilement adaptée au site à analyser, notamment en fonction de sa profondeur moyenne et du marnage local.

A cet effet, l'invention propose une station de mesure de données et/ou de collecte de bio-salissures dans un milieu sous-marin, caractérisée en ce qu'elle comprend:
- un châssis modulaire comprenant au moins deux modules rigides superposés selon un axe sensiblement vertical et destinés à être immergés en continu dans le milieu sous-marin, lesdits modules étant configurés pour définir un volume, dit volume intérieur, à l'intérieur dudit châssis,
- une embase, formant lest, à laquelle est attaché le module le plus bas du châssis, ladite embase étant destinée à reposer sur le sol sous-marin,
- des capteurs de mesure de données et/ou des dispositifs de collecte de bio-salissures montés sur au moins un module du châssis,
- au moins une bouée centrale destinée à être immergée en continu et reliée par des câbles à au moins l'un desdits au moins des deux modules ou à l'embase, éventuellement via les dispositifs de collecte, de sorte que lesdits câbles soient présents dans ledit volume intérieur, ladite bouée centrale étant destinée à tendre lesdits câbles de manière à assurer la stabilité et/ou la verticalité de ladite station.

Selon l'invention, la structure de la station étant modulaire, elle peut être montée ou démontée facilement, être transportée aisément sur site. Elle peut être également retirée sans difficulté du milieu sous-marin afin de récupérer les dispositifs de collecte de bio-salissures ou les capteurs de mesure. Cette structure modulaire permet également d'adapter le nombre de modules au site à étudier, notamment à sa profondeur et à la typologie potentielle de bio-colonisation.

Ce châssis modulaire avec superposition de modules permet également de disposer de mesures par strates d'eau tout en limitant la contamination des strates entre elles.

La bouée centrale participe avec le lest à améliorer la stabilité et la verticalité de la station en tendant les câbles attachés aux modules. Elle confère une certaine flottabilité à la structure, facilite sa mise en place et assure la prétention des câbles donc la stabilité d'ensemble, ce qui participe également à faciliter son retrait du milieu sous-marin et son transport.

Selon un mode de réalisation particulier, la bouée centrale et lesdits câbles sont agencés pour que ladite au moins une bouée centrale soit également présente dans ledit volume intérieur. En variante, la bouée centrale est positionnée au-dessus du châssis, seuls les câbles la reliant aux modules étant disposés dans le volume intérieur.

Selon un mode de réalisation particulier, la bouée centrale est apte à être ballastée au moment de l'installation de la station de mesure sur le sol sous-marin. Elle est dé-ballastée au moment du démantèlement de la station. Ce ballastage de la bouée centrale participe à améliorer encore la stabilité de la station.

Si la station, de par son concept et sa constitution, permet de mesurer la bio-colonisation, elle permet également de mesurer d'autres phénomènes comme la corrosion ou la pénétration des ions chlorures dans un matériau poreux. La corrosion peut être par exemple mesurée sur les montants des modules et la dégradation du béton est mesurée en analysant l'embase.

Selon un mode de réalisation particulier, chaque module est un cadre rigide comprenant au moins trois montants reliés par des traverses, lesdits montants et traverses étant agencés pour définir un volume dans lequel les câbles et, le cas échéant, ladite bouée centrale sont disposés. Cette simplicité de la structure permet d'obtenir un module léger.

Selon un mode de réalisation particulier, le châssis comporte une portion dite immergée comprenant lesdits modules immergés en continu et, au-dessus de ladite portion immergée, une portion dite de marnage comprenant au moins un module rigide destiné à être périodiquement émergé. Ce mode de réalisation est employé pour faire des mesures dans des zones de marnage et assurer également la localisation visuelle de la station pour les autres acteurs travaillant dans la zone (pêche, tourisme, ...). Cette portion de marnage permet également de fixer des dispositifs d'alimentation électrique pour les capteurs, par exemple un panneau solaire ou une éolienne.

Dans ce mode de réalisation, la portion immergée du châssis comporte avantageusement, en partie supérieure, un module rigide équipé d'un flotteur sur chacun de ses montants, lesdits flotteurs étant disposés à même hauteur sur les montants pour maintenir la verticalité de la station contre les efforts extérieurs environnementaux s'exerçant sur la portion de marnage et pour assurer la flottabilité de la station en compensant en particulier le poids de la portion de marnage de la station.

Selon un mode de réalisation particulier, la section transversale du cadre rigide des modules est triangulaire. Selon des modes de réalisation en variante, la section transversale des modules est polygonale, elliptique ou circulaire.

Selon un mode de réalisation particulier, l'embase comporte avantageusement des compartiments de ballastage aptes à être remplis d'eau de mer, pour faciliter le transport par flottaison, l'installation et le démantèlement de la station.

Selon un mode de réalisation particulier, les capteurs de mesure et/ou les dispositifs de collecte de bio-salissures sont fixés aux montants du module. Le nombre de capteurs de mesure et de dispositifs de collecte de bio-salissures et la nature des capteurs est variable.

Selon un mode de réalisation particulier, les dispositifs de collecte de bio-salissures sont tubulaires afin de respecter les formes usuelles qui influent sur l'écoulement et donc sur la fixation du biofilm ou les phénomènes d'abrasion. Selon un mode de réalisation, les capteurs de mesure sont, pour au moins une partie d'entre eux, disposés dans le tube des dispositifs de collecte de bio-salissures pour limiter les risques de détérioration par des chocs ou des frottements. Selon un mode de réalisation particulier, les capteurs de mesure de données et/ou les dispositifs de collecte sont amovibles.

D'autres avantages pourront encore apparaître à l'homme du métier à la lecture des exemples ci-dessous, illustrés par les figures annexées, donnés à titre illustratif.

### Brève description des figures

- La figure 1 représente une vue schématique en perspective d'une station de mesure selon un premier mode de réalisation de l'invention;
- La figure 2 représente une vue schématique d'un module du châssis de la station de mesure de la figure 1;
- La figure 3 représente une vue schématique du module de la figure 2, dans lequel les dispositifs de collecte des bio-salissures ont été retirés;
- La figure 4 représente une vue schématique en perspective d'une station de mesure selon un deuxième mode de réalisation de l'invention;
- La figure 5 représente une vue schématique en perspective d'une station de mesure selon un troisième mode de réalisation de l'invention; et
- La figure 6 représente une vue schématique en perspective d'une station de mesure selon un quatrième mode de réalisation de l'invention.

### Description détaillée de l'invention

Selon l'invention, il est proposé une station de mesure de données et de collecte de bio-salissures destinée à être installée sur un site sous-marin pour recueillir des données sur la dégradation et/ou la bio-colonisation du site ainsi que certaines données environnementales, si possible quelques années avant l'exploitation dudit site, par exemple avant l'installation d'une structure offshore telle qu'une hydrolienne sur ce site.

Cette station de mesure est implantée sur le site avant installation de la structure offshore pour identifier et mesurer la colonisation des organismes marins et/ou les mécanismes de dégradation présents sur le site. Cette identification pourra éventuellement orienter les derniers choix de conceptions de la structure offshore.

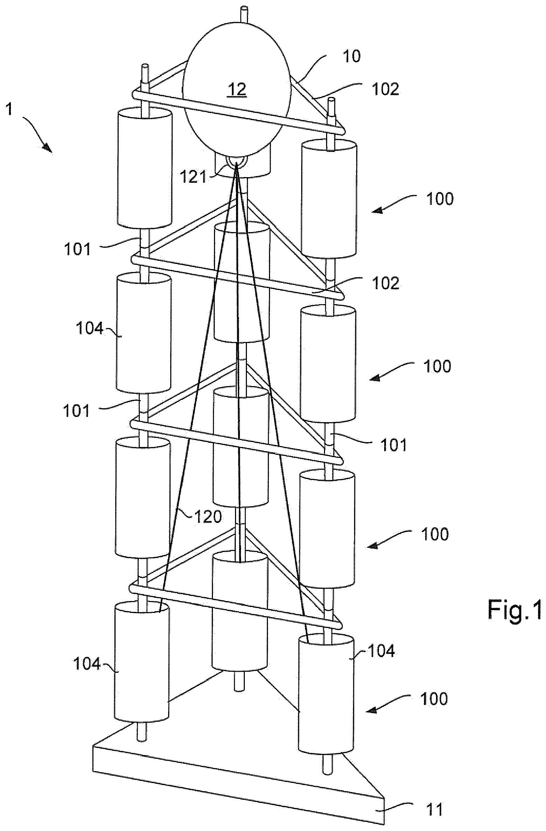

En référence à la figure 1, la station de mesure 1 comprend un châssis 10 modulaire et une embase 11 pesante formant lest destinée à reposer sur le sol sous-marin du site à étudier.

Le châssis comprend quatre modules rigides 100 superposés selon un axe sensiblement vertical. Ces modules sont destinés à être immergés en continu dans le milieu sous-marin. Chaque module 100 comprend trois montants 101 reliés par trois traverses 102 de manière à former un cadre rigide de section transversale triangulaire.

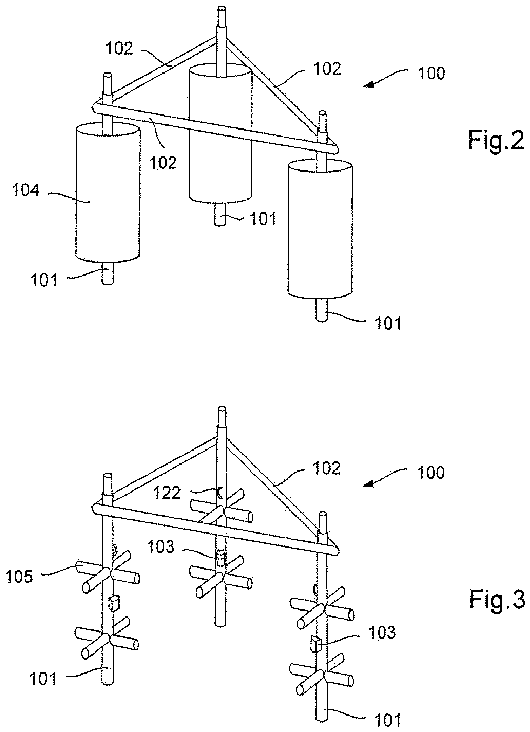

Comme illustré aux figures 2 et 3, les montants 101 sont reliés entre eux à proximité de leur extrémité supérieure par les traverses 102. Les montants et les traverses sont des tubes creux en matière inoxydable. Les traverses peuvent être reliées entre elles pour former un cadre triangulaire comme dans l'exemple illustré ici. Un montant est fixé, par exemple par soudage, à chaque angle du cadre formé par les traverses, perpendiculairement à celui-ci.

Dans le mode de réalisation illustré ici, chaque montant 101 présente, à son extrémité supérieure, un diamètre réduit pour pourvoir l'enfoncer dans l'extrémité inférieure d'un montant d'un module disposé au-dessus de lui. Des moyens de blocage, non représentés, sont prévus pour bloquer en translation les deux modules. Ces moyens de blocage sont par exemple constitués d'un pion élastique monté sur la paroi de l'extrémité supérieure des montants du module inférieur et apte à venir s'engager dans un orifice ménagé dans la paroi de l'extrémité inférieure des montants du module supérieur.

En variante, un assemblage par anneaux-mousquetons-câbles est utilisé pour attacher ensemble les montants de deux modules superposés.

L'embase 11 est par exemple un bloc de béton également équipé de trois tubes sensiblement verticaux dont l'extrémité supérieure est destinée à venir s'emmancher dans l'extrémité inférieure du module le plus bas du châssis 10. Les mêmes moyens de blocage peuvent être prévus pour attacher ledit module à l'embase.

Avantageusement, l'embase comporte des compartiments de ballastage, à la place ou en complément du bloc de béton, lesdits compartiments de ballastages étant aptes à être remplis d'eau de mer une fois la station amenée sur site. Ceci permet de faciliter le transport sur site de la station par flottaison, son installation et, une fois la mission terminée, son démantèlement.

Avantageusement, l'embase 11 sert également à mesurer les mécanismes de dégradation du béton, notamment par des bactéries marines, et le taux de pénétration d'ions chlorure dans le béton.

Les modules 100 sont équipés de capteurs de mesure de données 103 et/ou des dispositifs de collecte de bio-salissures 104.

Les capteurs de mesure 103 mesurent par exemple les conditions océanographiques et les caractéristiques physico-chimiques d'eau du site. Les capteurs sont par exemple des capteurs de température, des mini-caméras, des capteurs de mesure d'oxygène dissous... Les mesures peuvent être réalisées par strates, chaque module de la station correspondant alors à une strate.

Les dispositifs de collecte de bio-salissures 104 sont par exemple des dispositifs tubulaires fixés par des éléments de fixation en croix 105 aux montants 101.

Dans le mode de réalisation présenté ici, les capteurs de mesure 103 sont fixés aux montants et disposés à l'intérieur des tubes constituant les dispositifs de collecte 104 pour les protéger des chocs et/ou des frottements avec des corps étrangers. Avantageusement, des éléments de protection, sous forme de grilles amovibles, sont montés sur chacune des extrémités des tubes afin de protéger les capteurs contre certains sédiments et bio-salissures.

Bien entendu, les capteurs de mesure peuvent aussi être fixés aux traverses 102 ou sur la face extérieure ou intérieure des dispositifs de collecte 104.

Les capteurs de mesure 103 et/ou les dispositifs de collecte 104 sont de préférence fixés de manière amovible au châssis afin d'être retirés facilement du châssis.

Selon une caractéristique importante de l'invention, la station comporte au moins une bouée centrale 12 reliée par des câbles 120 à au moins l'un des modules 100 et/ou à l'embase 11, laquelle bouée est destinée à améliorer la verticalité et la stabilité de la station.

Les câbles 120 et, éventuellement la bouée 12, sont disposés à l'intérieur du volume, dit volume intérieur, défini par le châssis. Dans l'exemple de la figure 1, la bouée centrale est partiellement disposée dans le volume intérieur du châssis. En variante, elle peut être disposée en totalité dans ledit volume intérieur ou être disposée au dessus du châssis. Dans ce dernier cas, seuls les câbles sont présents dans le volume intérieur du châssis.

La bouée centrale 12 a pour fonction de tendre les câbles de manière à améliorer la verticalité de ladite station et la flottabilité des modules. Elle est par exemple de forme générale sphérique ou ellipsoïdale.

Les câbles 120 sont reliés, par exemple au moyen de mousquetons (non représentés), par une de leurs extrémités à une boucle 121 de la bouée centrale et par l'autre extrémité à un anneau 122 fixé aux montants 101.

Avantageusement, la bouée centrale est ballastée au moment de l'installation de la station de mesure sur le sol sous-marin et dé-ballastée au moment de son démantèlement. Ce ballastage contribue à augmenter la stabilité de la station.

Cette architecture de station offre de nombreux avantages:
- le châssis étant modulaire, elle peut être démontée et remontée simplement et retirée facilement du milieu sous-marin pour récupérer les dispositifs de collecte de bio-salissures ou les capteurs de mesure;
- le châssis étant réalisé avec de simples tubes, elle est légère et peut donc être transportée facilement sur site;
- la présence de la bouée centrale confère une certaine stabilité (verticale) au châssis par poussée d'Archimède, laquelle poussée met en tension les câbles du dispositif grâce à la présence, à l'extrémité basse du dispositif, du lest (embase 11);
- l'architecture modulaire du châssis permet d'adapter le nombre de modules au site à étudier, notamment à sa profondeur.

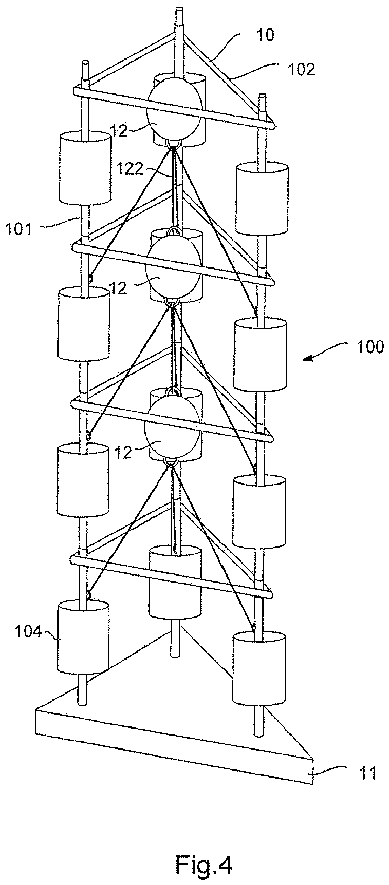

La station peut comporter une ou plusieurs bouées centrales 12 pour conférer de la stabilité à la structure. Dans le mode de réalisation illustré par la figure 4, la station comporte trois bouées centrales 12 reliées à trois modules 100 distincts. Le nombre et la taille des bouées centrales dépendent de nombreux paramètres et notamment du volume intérieur du châssis, de la hauteur et du poids du châssis et du poids de l'embase.

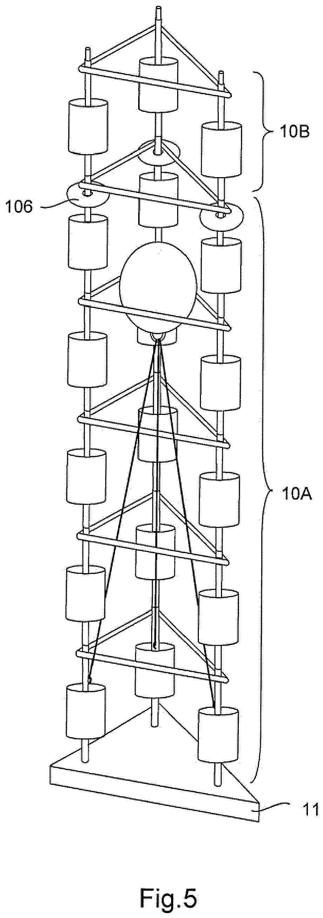

Si la station comprend plusieurs bouées centrales 12, ces dernières sont avantageusement reliées ensemble par un câble dédié 122 de manière à corréler leurs déplacements. Chaque bouée centrale peut aussi être attachée à plusieurs modules avec des longueurs de câbles adaptées pour contraindre le châssis à différentes hauteurs. Si le dispositif comporte une seule bouée, celle-ci peut être reliée par les câbles 120 à un seul module, qui peut être le module inférieur de la portion immergée comme montré sur les figures 1 et 5, un module intermédiaire ou le module supérieur, ou être reliée à plusieurs modules.

Dans les modes de réalisation illustrés par les figures 1 et 4, les modules 100 du châssis sont destinés à être immergés en continu dans le milieu sous-marin.

Dans un autre mode de réalisation illustré par la figure 5, le châssis de la station comporte des modules destinés à être immergés en continu et au moins un module supplémentaire, en partie supérieure du châssis, destiné à être périodiquement émergé lorsque la marée en basse. Le châssis 10 comporte donc une portion immergée référencée 10A comprenant les modules destinés à être immergés en continu et une portion référencée 10B, appelée portion de marnage, comprenant le ou les modules destinés à être périodiquement émergés.

Le module émergé périodiquement est de préférence identique aux modules immergés en continu pour simplifier la fabrication. Il peut servir de support à des dispositifs destinés à alimenter en énergie électrique les capteurs, par exemple un panneau solaire ou une éolienne.

Dans ce mode de réalisation (figure 5), le module le plus haut de la portion immergée 10A comporte avantageusement un flotteur 106 sur chacun de ses montants 101 pour améliorer la stabilité de la station et assurer sa flottabilité en supportant le poids du module émergé.

Les flotteurs 106 sont disposés à même hauteur sur les montants et sont positionnés pour rester immergés quelle soit la hauteur de la marée basse (cette hauteur varie selon le coefficient de marée) de manière à ce que la station conserve en permanence la même stabilité. Ces flotteurs positionnés hauts sur le châssis ont pour rôle de s'opposer aux efforts exercés par le vent et la houle sur la portion de marnage 10B de la station.

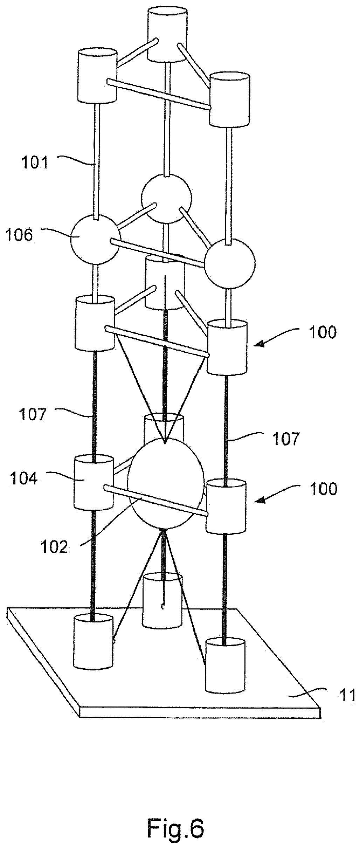

La figure 6 illustre un autre mode de réalisation de l'invention. L'embase référencée 11 est de forme rectangulaire et les flotteurs 106 sont des ballons. La portion immergée comporte deux modules 100. Chacun de ces modules comporte une structure tubulaire en forme de triangle. Un dispositif de collecte 104 est placé à chacun des sommets du triangle. Les montants rigides sont replacés par des câbles ou des chaines 107 et les flotteurs 106 servent alors à tendre les câbles ou chaines 107. Le module 100 le plus bas est aussi rattaché à l'embase 11 par des câbles ou chaines.

Les modes de réalisation décrits ci-dessus ont été donnés à titre d'exemple. Il est évident pour l'homme de l'art qu'ils peuvent être modifiés, notamment quant au nombre ou à la forme des modules, quant au nombre ou à la nature des capteurs de mesure, quant au nombre ou à la forme des dispositifs de collecte de bio-salissures, quant au nombre, la forme ou la disposition des bouées centrales, quant à la forme de l'embase et quant à la nature du lest utilisée dans l'embase.

## Revendications

1. Station (1) de mesure de données et/ou de collecte de bio-salissures dans un milieu sous-marin, **caractérisée en ce qu'**elle comprend:
- un châssis (10) modulaire comprenant au moins deux modules (100) rigides superposés selon un axe sensiblement vertical et destinés à être immergés en continu dans le milieu sous-marin, lesdits modules étant configurés pour définir un volume, dit volume intérieur, à l'intérieur dudit châssis,
- une embase (11), formant lest, à laquelle est attaché le module le plus bas du châssis, ladite embase étant destinée à reposer sur le sol sous-marin,
- des capteurs de mesure (103) de données et/ou des dispositifs de collecte (104) de bio-salissures montés sur au moins un module du châssis,
- au moins une bouée centrale (12) destinée à être immergée en continu et reliée par des câbles (120) à au moins l'un desdits au moins des deux modules (100) et/ou à l'embase de sorte que lesdits câbles soient présents dans ledit volume intérieur, ladite bouée centrale étant destinée à tendre lesdits câbles de manière à assurer la stabilité et/ou la verticalité de ladite station.

2. Station selon la revendication 1, **caractérisée en ce que** ladite au moins une bouée centrale et lesdits câbles sont agencés pour que ladite au moins une bouée centrale soit présente dans ledit volume intérieur.

3. Station selon la revendication 1 ou 2, **caractérisée en ce que** chaque module (100) est un cadre rigide comprenant au moins trois montants (101) reliés par des traverses (102), lesdits montants et traverses étant agencés pour définir un volume dans lequel lesdits câbles et, le cas échéant, ladite au moins une bouée centrale (12) sont disposés.

4. Station selon la revendication 3, **caractérisée en ce que** le châssis comporte une portion dite immergée (10A) comprenant lesdits modules immergés en continu et, au-dessus de ladite portion immergée, une portion (10B) dite de marnage comprenant au moins un module rigide destinés à être périodiquement émergé.

5. Station selon la revendication 4, **caractérisée en ce que** la portion immergée du châssis comporte, en partie supérieure, un module rigide équipé d'un flotteur (106) sur chacun de ses montants, lesdits flotteurs étant disposés à même hauteur sur les montants pour assurer la flottabilité et la verticalité de la station contre les efforts extérieurs s'exerçant sur la portion de marnage de la station.

6. Station selon l'une quelconque des revendications précédentes, **caractérisée en ce que** l'embase comporte des compartiments de ballastage aptes à être remplis d'eau de mer.

7. Station selon l'une quelconque des revendications précédentes, **caractérisée en ce que** la section transversale du cadre rigide des modules est triangulaire.

8. Station selon l'une quelconque des revendications précédentes, **caractérisée en ce que** les capteurs de mesure (103) et/ou les dispositifs de collecte (104) de bio-salissures sont fixés aux montants (101) du module.

9. Station selon l'une quelconque des revendications précédentes, **caractérisée en ce que** les dispositifs de collecte (104) de bio-salissures sont tubulaires.

10. Station selon la revendication 6, **caractérisé en ce que** des capteurs de mesure (103) sont disposés dans le tube des dispositifs de collecte (104) de bio-salissures.

11. Station selon l'une quelconque des revendications précédentes, **caractérisée en ce que** les capteurs de mesure (103) de données et/ou les dispositifs de collecte (104) sont amovibles.

## Patentansprüche

1. Station (1) zum Messen von Daten und/oder zum Sammeln von biologischen Verschmutzungen in einem Meeresmilieu, **dadurch gekennzeichnet, dass** sie umfasst:
- ein modulares Gestell (10), das mindestens zwei starre Module (100) umfasst, die gemäß einer im Wesentlichen vertikalen Achse übereinander angeordnet und dazu bestimmt sind, kontinuierlich im Meeresmilieu untergetaucht zu sein, wobei die Module so ausgebildet sind, dass sie ein als inneres Volumen bezeichnetes Volumen im Inneren des Gestells definieren,
- einen Sockel (11), der einen Ballast bildet, an dem das unterste Modul des Gestells festgemacht ist, wobei der Sockel dazu bestimmt ist, auf dem Meeresboden zu ruhen,
- Sensoren (103) zum Messen von Daten und/oder Vorrichtungen (104) zum Sammeln von biologischen Verschmutzungen, die an mindestens einem Modul des Gestells montiert sind,
- mindestens eine zentrale Boje (12), die dazu bestimmt ist, kontinuierlich untergetaucht zu sein und die über Kabel (120) derart mit mindestens einem der mindestens zwei Module (100) und/oder mit dem Sockel verbunden ist, dass sich die Kabel in dem inneren Volumen befinden, wobei die zentrale Boje dazu bestimmt ist, die Kabel zu spannen, um die Stabilität und/oder die Vertikalität der Station sicherzustellen.

2. Station gemäß Anspruch 1, **dadurch gekennzeichnet, dass** die mindestens eine zentrale Boje und die Kabel so eingerichtet sind, dass sich die mindestens eine zentrale Boje in dem inneren Volumen befindet.

3. Station gemäß Anspruch 1 oder 2, **dadurch gekennzeichnet, dass** jedes Modul (100) ein starrer Rahmen ist, der mindestens drei Streben (101) umfasst, die über Querträger (102) verbunden sind, wobei die Streben und Querträger so eingerichtet sind, dass sie ein Volumen definieren, in dem die Kabel und gegebenenfalls die mindestens eine zentrale Boje (12) angeordnet sind.

4. Station gemäß Anspruch 3, **dadurch gekennzeichnet, dass** das Gestell einen so genannten untergetauchten Abschnitt (10A) umfasst, der die kontinuierlich untergetauchten Module umfasst, und oberhalb des untergetauchten Abschnitts einen so genannten Tidenhubabschnitt (10B), der mindestens ein starres Modul umfasst, das dazu bestimmt ist, regelmäßig aufgetaucht zu werden.

5. Station gemäß Anspruch 4, **dadurch gekennzeichnet, dass** der untergetauchte Abschnitt des Gestells im oberen Teil ein starres Modul umfasst, das an jedem seiner Streben mit einem Auftriebskörper (106) ausgestattet ist, wobei die Auftriebskörper an den Streben auf gleicher Höhe angeordnet sind, um den Auftrieb und die Vertikalität der Station entgegen der äußeren Kräfte, die auf den Tidenhubabschnitt der Station einwirken, sicherzustellen.

6. Station gemäß einem der vorstehenden Ansprüche, **dadurch gekennzeichnet, dass** der Sockel Ballastkammern umfasst, die dazu geeignet sind, mit Meerwasser gefüllt zu werden.

7. Station gemäß einem der vorstehenden Ansprüche, **dadurch gekennzeichnet, dass** der Querschnitt des starren Rahmens der Module dreieckig ist.

8. Station gemäß einem der vorstehenden Ansprüche, **dadurch gekennzeichnet, dass** die Messsensoren (103) und/oder die Vorrichtungen (104) zum Sammeln von biologischen Verschmutzungen an den Streben (101) des Moduls befestigt sind.

9. Station gemäß einem der vorstehenden Ansprüche, **dadurch gekennzeichnet, dass** die Vorrichtungen (104) zum Sammeln von biologischen Verschmutzungen röhrenförmig sind.

10. Station gemäß Anspruch 6, **dadurch gekennzeichnet, dass** in dem Rohr der Vorrichtungen (104) zum Sammeln von biologischen Verschmutzungen Messsensoren (103) angeordnet sind.

11. Station gemäß einem der vorstehenden Ansprüche, **dadurch gekennzeichnet, dass** die Sensoren (103) zum Messen von Daten und/oder die Sammelvorrichtungen (104) abnehmbar sind.

## Claims

1. A station (1) for measuring data and/or collection of biofouling in an undersea environment, **characterized in that** it comprises:
- a modular frame (10) comprising at least two rigid modules (100) superimposed along a substantially vertical axis and intended to be immersed continuously into the undersea environment, said modules being configured to define a volume, called inner volume, within said frame,
- a base (11) forming ballast, to which is attached the lower modulus of the frame, said base being intended to rest on the seabed,
- measuring sensors (103) of data and/or biofouling collection devices (104) mounted on at least one module of the frame,
- at least one central buoy (12) intended to be continuously immersed and connected by cables (120) to at least one of said at least two modules (100) and/or to the base so that said cables are present in said inner volume, said central buoy being for tensioning said cables so as to ensure the stability and/or the verticality of said station.

2. A station according to claim 1, **characterized in that** said at least one central buoy and said cables are arranged so that said at least one central buoy is present in said inner volume.

3. A station according to claim 1 or 2, **characterized in that** each module (100) is a rigid housing comprising at least three uprights (101) connected by crosspieces (102), said uprights and crosspieces being arranged to define a volume wherein said cables and, where appropriate, said at least one central buoy (12) are arranged.

4. A station according to claim 3, **characterized in that** the frame comprises a so-called submerged portion (10A) comprising said continuously immersed modules and, above said immersed portion, a portion (10B), called tidal range portion, comprising at least one rigid module to be periodically emerged.

5. A station according to claim 4, **characterized in that** the submerged portion of the frame comprises, at the top, a rigid module with a float (106) on each of its uprights, said floats being disposed at the same height on the uprights to provide buoyancy and verticality to the station despite the external forces exerted on the tidal range portion of the station.

6. A station according to any one of the preceding claims, **characterized in that** the base comprises ballast compartments adapted to be filled with sea water.

7. A station according to any one of the preceding claims, **characterized in that** the crosspiece of the rigid frame of the modules is triangular.

8. A station according to any one of the preceding claims, **characterized in that** the measuring sensors (103) and/or biofouling collection devices (104) are fixed to the uprights (101) of the module.

9. A station according to any one of the preceding claims, **characterized in that** the biofouling collection devices (104) are tubular.

10. A station according to claim 6, **characterized in that** the measuring sensors (103) are arranged in the tube of the biofouling collection devices (104).

11. A station according to any one of the preceding claims, **characterized in that** the measuring sensors (103) of data and/or collection devices (104) are removable.
